# EUROPEAN PATENT APPLICATION

(11) **EP 1 224 903 A1**
(43) Date of publication of application: **24.07.2002**
(21) Application number: 01100892.7
(22) Date of filing: 16.01.2001
(51) Int. Cl.: A61B 5/00, A61J 7/04, G06F 19/00, B42D 15/00

(54) **A system and a method for monitoring the effectiveness of a medical treatment**

(71) Applicant: Microlife Intellectual Property GmbH, 9442 Berneck (CH)
(72) Inventor: Lin, Wen-Guai, Sec.6, Min-Chuan East Road, Taipei (TW); Fong, Yi-Ling, Shan-Chung City, Taipei (TW); Frick, Gerhard, 6800 Feldkirch (AT)
(74) Representative: Hepp, Dieter

(57) **Abstract**

In a system for monitoring the effectiveness of a medical treatment of a patient, signals indicating whether the patient (online) with a medicine regimen and with a health parameter measurement schedule and health parameter measurement data are printed out on a printer (13, 23) or displayed on other display means (33). The doctor therefore may easily monitor the influence of a medication to the health parameters.

## Description

The invention relates to a system and to a method for monitoring the effectiveness of a medical treatment.

The need for monitoring of medication compliance has been widely recognized. In America, about 125,000 persons die annually because they did not take their medicine as prescribed (source: Anderson, Update on patient non-compliance). It is also recognized that continuous monitoring of health parameters such as blood pressure, glucose level or body fat are important in view of the control of a medical treatment.

In the European patent application EP-301 672A2 an interactive drug dispenser has been proposed. The drug dispenser actively controls the manner in which medicine is administered to a patient.

EP-370 599B1 discloses a medicine container which is provided with a drug dispensing event detector. Removal of medicine from the container is recorded by detection of cap openings of the container.

US-4 933 873 discloses an interactive patient assistant device, which includes both preselected doses of medication and a physical testing device. The device keeps track of medication and diagnostic testing schedules. The device makes a preselected dose of medication available to the patient. The system can communicate with a remote medical center over the telephone system by way of a modem.

US-5 016 172 discloses a system for monitoring patient compliance and patient status. Medicine consumption as well as physical parameter such as heart rate or the like are recorded. Those parameters are transmitted from time to time to a central computer and optionally to a remote computer for professional or hospital use.

US-5 710 551 discloses a system for remote monitoring of self-medication of a patient to assure compliance with a prescribed dosage schedule. The system may be connected to a remote central monitoring station via a communications link.

WO98/49659 discloses a prescription compliance device and method. A patient is reminded when the next dose of medication is to be taken and indicates whether a specified dose has been taken.

WO00/32088 discloses a medical system and a method of controlling the system for medical self-treatment. The system consists of several portable modules. One module is designated as a master module, which controls, supervises and monitors information and data exchange between itself and the rest of the modules. The modules may consist of a measuring device (blood glucose monitor) or of a doser, an inhaler, a tablet dispenser and storage container.

WO00/32097 discloses a system and a method for executing a treatment regimen. Medical personnel can receive feedback from the patient regarding the medical regimen. A medicine dispenser is coupled to a client device. The medicine dispenser dispenses only the dosage of medicine directed by this prescribed medical regimen and records information from the patient regarding compliance.

US-5 967 975 discloses a system for the automated remote monitoring of home health parameter measurement activities to assure outpatient subscriber compliance with a prescribed measurement schedule regimen. The monitored health parameters typically included blood pressure, blood glucose level, clotting factor and the like.

Despite the large number of systems for monitoring patient compliance, no such devices nor systems are widely used in current health care systems. One drawback of the systems proposed in the prior art is that they are relatively complicated to use or to install. Especially, many of the proposed systems rely on communication links which need computers both at the patient's and at doctor's or health care provider's location. Most existing health insurance reimbursement systems do not provide any incentive to the patient and to the doctor to install such complicated and expensive systems and related operating software platforms. Such systems therefore are, although described in theory, not used in practice.

In addition, such systems try to solve the compliance problem by remotely monitoring the patient without human contact between the patient and the doctor. Doctors are reluctant to use such monitoring and there is therefore a resistance to adopt such known systems in the real medical world.

The known systems rely on monitoring of patient compliance with a medication regimen or with a health parameter measuring schedule. The known systems and methods therefore do not allow to directly monitor the effectiveness of a medical treatment of a patient depending on patient compliance with a medication regimen.

It is therefore an object of the present invention to overcome the drawbacks of the prior art. Especially, it is an object of the present invention to provide a system and a method for monitoring the effectiveness of a medical treatment of a patient which has a simple design, which is manufacturable in an economic way and which therefore is affordable for a maximum number of users. The way how the system works should be compatible to the traditional relationship between a patient and a doctor. But it should allow a higher degree of automation and the system should be less time consuming and more sure for both, the patient and the doctor. Especially, it should be easier for the patient to obtain the necessary information. In addition, comprehensive medical evidence should be provided by gathering a multitude of information including one or several health parameters and medication compliance. The system should not necessitate extra work for the doctor. Furthermore, the system shall be easy to use for both patient and medical care person.
According to the invention, these objects are solved with a system and with a method according to the features of the patent claims.

The system for monitoring the effectiveness of a medical treatment of a patient comprises a medicine container, at least one measuring device for measuring a health parameter of the patient, at least one control and calculating unit and a printer or other display means.

The medicine container is designed to produce a first signal which indicates when the patient has taken medicine. The first signal especially may be produced when the container is opened. While a container opening is not an absolute proof of a patient taking his medicine, this design provides an easy to use container which is reliable and which may be manufactured in an economic manner.

The measuring device is used to measure at least one health parameter of the patient. Typical health parameters are the heart rate, blood pressure values, blood glucose level, cholesterol level, body fat, body weight or the like. The at least one measuring device is used to produce a second signal which is indicative of the health parameter.

The first and the second signal provided by the medicine container and by the measuring device may be coupled in the control and calculating unit for storage or display of medication compliance data and/or of health parameter data.

The printer or the other display means is further adapted to be coupled to the control and calculating unit. Thereby, a series of medication compliance data and of health parameter data of the patient may be printed out with the printer or displayed on the display. The system according to the present invention has several advantages. First, medication compliance data and health parameter data may be printed out on a piece of paper before the patient sees her or his doctor. Consequently, problems existing with patients forgetting to collect medication compliance data or health parameter data are avoided and the time used by the patient for monitoring those data is reduced. Expensive installations such as computers, telecommunication links and costs of phone connections may therefore be dispensed with. The patient simply shows the print out of the system to the doctor. Especially, the doctor does not need any additional hardware or extra work. The print out provided by the patient can be simply added to the patient's file in the doctor's office.

Secondly, medication compliance data and health parameter data are printed out or displayed next to one another and may therefore be brought in correlation to each other. It is therefore possible for the doctor to immediately observe the influence of compliance or non compliance with a medication regimen to the measured health parameter. Effectiveness of the treatment may be easily monitored. Depending on the effectiveness, the treatment may be tuned by the doctor.

It is also possible to store the medication compliance data or the health parameter data of the patient in a memory of a portable device, such as a personal digital assistant (PDA) which may be brought to the doctor or in a portable measuring device such as a wrist type blood pressure monitor or in another portable electronic device. It is further possible to transmit the medication compliance data and the health parameter data which are correlated to each other e.g. from a PDA via a communications link to a central computer if such link is available.

This system may also include a memory, (e.g. associated with the control and calculating unit) in which a medication schedule and/or a health parameter measurement schedule is stored. Such schedules have several advantages: it is possible to remind the user to take its medicine or to perform a health parameter measurement if it is not made according to these schedules. It is also possible to visually indicate on the print out or on the display dates or times when the patient has complied or has not complied with the schedules.

Medication compliance data according to the present invention especially include data indicating whether the patient has taken its medicine or not. Medication compliance data may further include additional data such as time or day of compliance/non compliance or number of medication during a certain period of time or any other compliance data which is of medical relevance. Medicine means typically pills. Any other form of medicine (e.g in liquid form) may be used with an appropriate container.

Health parameter data typically include values of a measurement of the health parameter such as heart rate, systolic, diastolic or medium blood pressure etc. Health parameter data may also include time and date when the measurement has been made or also other relevant data.

It is possible to print out medication compliance data and health parameter data each time when the patient has complied or not complied with a medication compliance schedule or with a health parameter measurement schedule. With such a system, no storage of the data would be necessary. It is also conceivable and preferable to store the data for a certain period of time and to print out the data on a regular basis, e.g. once per week or to print out the data on request of the patient, e.g. before an appointment with his or her doctor.

According to a first embodiment of the invention, the system is formed as an integral unit. The unit has a housing which includes at the same time a medicine container, at least one measuring device and the printer. Such a system is especially suited for home use.

According to a second embodiment of the invention, the system comprises a base unit. The at least one measuring device is adapted to be connected to and removed from the base unit. It is also conceivable to provide a base unit where both, the medicine container and the measuring device may be connected to and removed from. Such systems is especially suitable for travel use. Measurement data may be stored in the base unit. When the patient is at home, the measurement data may be transmitted to a printer separate from the base unit. The patient therefore does not have to take the printer with him when travelling.
It is also possible to store the medicine compliance data in a medicine container and to store the health parameter data in the measuring device. In this case, the user does not even have to take the base unit with him. Data stored in the medicine container and in the measuring device may be subsequently transmitted to the base unit and/or to the printer.

It is preferred to provide the system with several connections to which a number of different health parameter measuring devices may be connected. Preferred is a mechanical connection (tube) for a cuff of a blood pressure measuring device and an infrared connection for any kind of measuring device such as weight scale, glucose meter or the like. The system with its at least one control and calculating unit plays the role of a central data collection center. In such a case, also several medicine containers may be used in order to distinguish different types of medicine.

According to a preferred embodiment of the invention, the system comprises means such as a print button for causing the printer to print out medication compliance data and health parameter data. According to the first embodiment of the invention, this print button may be arranged within the housing.

In the second embodiment of the invention, the print button may be arranged either on the printer separate from the base unit or on the base unit.

According to a further preferred embodiment of the invention, the system comprises a voice generator. The voice generator is used to remind the patient to take his or her medicine and/or to measure health parameters. For that purpose, the housing according to the first embodiment and the medicine container and/or the measuring device or the base unit of the second embodiment may be provided with a loud speaker.

The system according to the invention may be provided with storage means for storing a series of successive medication compliance data and a series of successive health parameter data. Storage of data allows also to make calculations on the basis of the data, such as calculating mean values of measured health parameters, percentages of compliance/non compliance or the like.

A series of successive medication compliance data/health parameter data according to the present invention means at least two indications whether the patient has complied with a medication schedule and at least two results of a measurement of a health parameter. Preferably, a substantial number of subsequent data such as data for a whole week or a whole month are stored in the storage means. When medication appliance data and health parameter data are stored in the system a log or a chart of a series of subsequent data may be calculated with the control and calculating unit. Such a log or chart may be printed out on the printer and may include a number of relevant information. Especially, compliance or non compliance with a medical schedule or a health parameter measurement schedule may be indicated with symbols. Corresponding times and measurement values may be printed out on a day to day basis.

Additional information such as a summary of the results, mean values, ranges of the measured health parameter values or a graphic indication of measurement results may be displayed.

The system according to the invention may further include a specifically designed sheet, onto which a number of print outs relating to different data may be attached. The sheet may be provided with adhesive tapes for such a purpose.

In the method according to the invention, a first signal indicative of a patient taking a medicine is generated. The first signal is transmitted to control and calculating unit. Medicine compliance data depending on said first signal are thereby stored or displayed.

In addition, at least one health parameter of the patient is measured. A second signal indicating this health parameter is generated. The second signal is transmitted to a control and calculating unit. Health parameter data depending on the second signal are stored or displayed thereby.

The steps of generating a first signal when a patient takes a medicine and of generating a second signal depending on the measurement of health parameters are repeated at least once. Thereby, a series of subsequent medicine compliance data and a series of successive health parameter data are generated. The series of successive medicine compliance data and health parameter data are subsequently displayed, especially printed out on a printer. The method according to the present invention allows to print out a series of successive medicine compliance data and health parameter data. A medical person such as a doctor therefore can easily verify effectiveness of a medical treatment.

According to a preferred embodiment of the method according to the invention, the medicine compliance data and the health parameter data are compared with a medicine compliance schedule and with a health parameter measurement schedule. The schedules are stored in a memory which may be associated to the control and calculating unit. In this case, especially health parameter data relating to the time of a health parameter measurement are compared with the parameter measurement schedule.

According to a further preferred embodiment of the method according to the invention, medicine compliance data and health parameter data may be transmitted to a printer. Transmission may be made via a communications link such as a wireless communication or through a telecommunication network. It is especially preferred, to transmit the data to a printer via an infrared communication.

According to a further preferred embodiment, voice messages for reminding the patient to comply with medication schedules or to measure health parameters may be generated.

With the method according to the invention, statistic values of the health parameter such as mean values and also statistic values relating to patient compliance data may be calculated and displayed upon demand.

According to still a further embodiment of the invention, compliance and non compliance with a medication schedule and/or with a scheduled health parameter measurement may be displayed with differently designed symbols. Such a print out allows a medical person to rapidly verify the influence of non compliance with a medicine regimen to the health parameters. Such an observation is especially easy when the measured health parameter values are graphically represented.

This invention will more fully understood by reference to the accompanying drawings, in which:
Figure 1 is a schematic representation of the first embodiment of a system according to the invention,
Figure 2 is a schematic representation of a second embodiment of a system according to the invention and
Figure 3 is a sample of a print out made with a system or with a method according to the present invention.
Figure 4 is a schematic representation of the system according to the invention,
Figure 5 is an example of a personal health passport and
Figures 6 and 7 are block diagramms of the first and second embodiment of the invention.

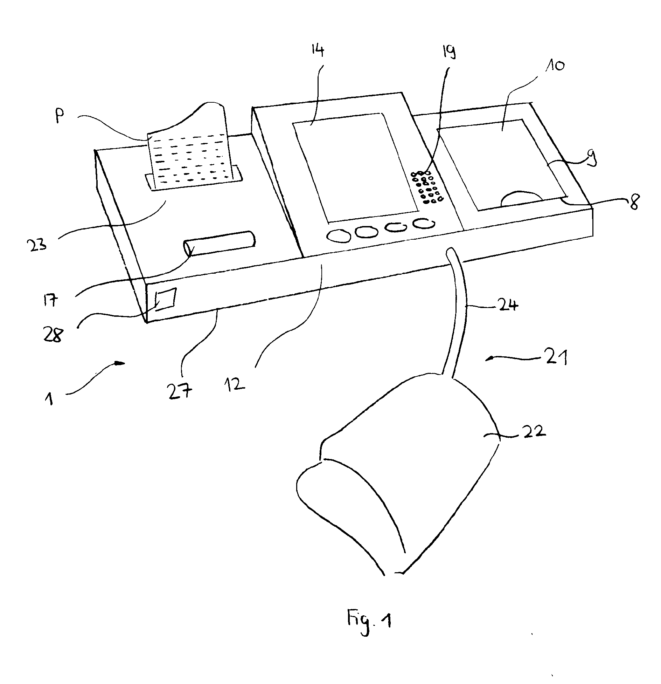

Figure 1 shows a system 1 for monitoring the effectiveness of a medical treatment of a patient. The system 1 comprises a container 10 in which medication such as pills is stored. The container 10 is closed with a cover 9. Cover 9 is provided with a switch 8 which registers opening of the cover 9.

The system is provided with a control and calculating unit 12. Signals produced by action of the switch 8 indicate, that the cover 9 has been opened. It is assumed that the patient has taken a prescribed medicine contained in the container 10, when the cover 9 is opened. Signals produced by switch 8 therefore indicate compliance of the patient with a medication regimen. It is conceivable to block operation of the switch for a certain period of time after activation.

The signal indicating compliance is transmitted to the control and calculating unit 12.

A blood pressure measuring unit 21 is also connected to the control and calculating unit 12. The blood pressure measuring unit 21 consists of a cuff 22 which is connected to the control and calculating unit 12 in a known manner with a tube 24. The blood pressure measuring unit 21 is a conventional upper arm type blood pressuring unit which works with the oscillometric method. For measuring the pressure within the cuff 22 and for inflating the cuff 22, the control and calculating unit 12 is provided with a pressure sensor, an automatic pump and valves in a way known to those skilled in the art. The control and calculating unit 12 determines the systolic and diastolic blood pressure measured with the blood pressure measuring unit 21 and may also determine the heart rate of the patient. Those values are displayed on a display 14.

The control and calculating unit 12 is further provided with a memory (not shown in Figure 1) for storing data relating to compliance for non compliance with a medication schedule or with a health parameter measurement schedule. In order to verify compliance or non compliance of the patient with a schedule, a medication schedule and a health parameter measurement schedule are also stored in a memory in the control and calculating unit 12.

Medication compliance data includes typically the information , whether or not the patient has complied with a medication regimen within a specific period of time defined by the medication schedule. Medical compliance data typically includes the information YES or NO and the time of compliance/non compliance.

Health parameter data typically include the values of the measured health parameters, e.g. the systolic and diastolic blood pressure and the heart rate in the embodiment according to Figure 1. Health parameter data further include information indicating whether or not the patient has complied with a health parameter measurement schedule and the date and time when the measurement schedule was complied with or non complied with. The system is provided within an infrared connection 28. Additional measuring devices such as a body weight scale, glucose or cholesterol meters may be connected to the control and calculating unit via the IR connection 28.

The system 1 further comprises a loud speaker 19. When the patient has not complied with a medication schedule or a health parameter measurement schedule within a certain period, he may be reminded to comply with the schedule by messages generated in a voice generator in the control and calculating unit 12 which are output with the loud speaker 19.

The system 1 is further provided with a printer 23. Medication compliance data and health parameter data may be printed out on a paper P when a printer button 17 is pressed. Typically a thermo-printer is used.

The system 1 according to the embodiment shown in Figure 1 is designed as a system for home use. The container 10, the control and calculating unit 12 with the display 14 and the printer 23 are arranged in a housing 27. The health parameter measuring device (blood pressure measuring unit 21 in Figure 1) is connected to the housing 27 with an appropriate connection (a tube 24 in Figure 1).

A number of variations of the embodiment shown in Figure 1 are conceivable.

Instead of a blood pressure monitor, other measuring devices for measuring health parameters may be used, such as glucose meter, cholesterol meters, weight scales or the like. Instead of the tube 24, appropriate electric connections will be used.

Instead of a switch 8 for registering container openings, it is also conceivable to use other detection means, such as weight dependent sensors which sense the weight of pills contained in the container 10 or light sensors which detect movements of the cover 9.

Instead of storing a series of medication compliance data and of health parameter data, it is also conceivable to immediately print out those data when a health parameter measurement has been made or when the patient has complied/not complied with a medication or a health parameter measurement scheduled.

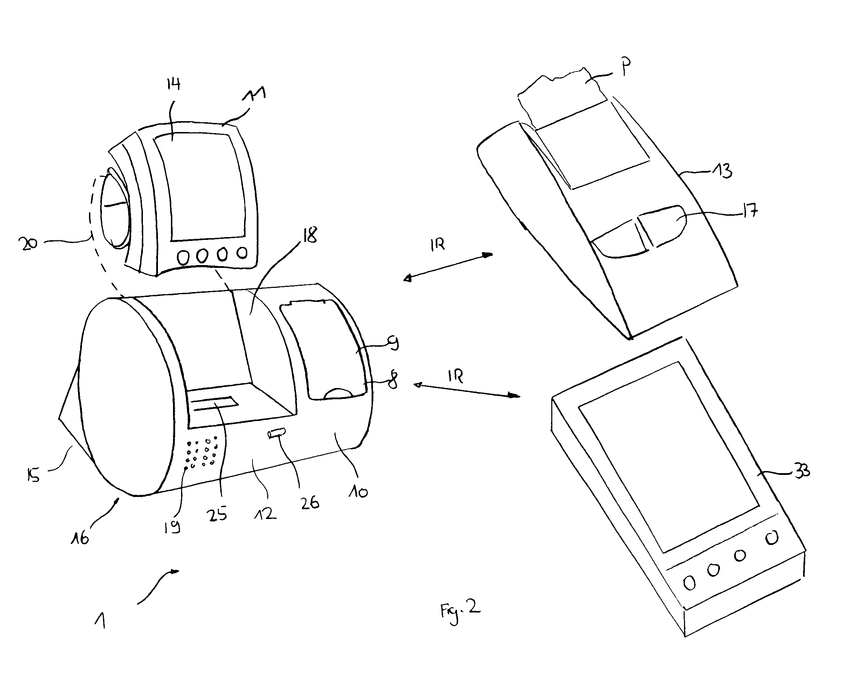

Figure 2 discloses another embodiment of the present invention. The system 1 shown in Figure 2 is especially suited for travel use.

The system 1 substantially consists of a base unit 16 and of a printer 13 and/or a personal digital assistant 33. The printer 13 or the personal digital assistant 33 may be connected with an infrared communications link to the base unit 16.

The base unit 16 comprises housing 15. The housing 15 is sized and shaped such that it is easily portable. The housing 15 includes a container 10 for medication. The container 10 is closed with a cover 9. Openings of the cover 9 are registered with a switch 8. The system also includes a blood pressure monitor 11. The blood pressure monitor 11 is formed as a known wrist type blood pressure monitor. The blood pressure monitor 11 comprises a display 14 on which health parameters such as blood pressure values and heart rate may be displayed. The blood pressure monitor 11 is stored in a spacing 18 within the housing 16. The spacing 18 may be closed with a cover 20 (only schematically shown in Figure 2).

In a similar way as in the embodiment according to Figure 1, compliance of the patient with the medication schedule is detected by means of container openings. Medication compliance data are stored in the control and calculating unit 12 which is also integral with the base unit 16.

For measuring its health parameters, the patient removes the blood pressure measuring device 11 from the spacing 18 and applies it around his wrist. When the measurement is terminated, the systolic and diastolic blood pressure values and the heart rate are displayed on the display 14 and are temporarily stored in a memory arranged within the blood pressure monitor 11. The base unit 16 is provided with an interface connection 25. The blood pressure monitor is provided with a mating interface connection (not shown in Figure 2). When the blood pressure monitor 11 is put in the spacing 18, a connection between the interfaces of the base unit 16 and of the blood pressure monitor 11 is established and health parameter data stored in the blood pressure monitor 11 are transferred to the base unit 16 and stored in a memory contained therein.

As the base unit 16 is portable, it is especially suitable for travel use. As medication compliance data and health parameter data are stored in the base unit 16, it is not necessary for the patient to take a printer with him.

The base unit 16 is further provided with an eject knob which allows removal of the blood pressure monitor 11 from the spacing 18. Eject knob 26 allows to hold the blood pressure monitor 11 firmly within the base unit 16 and allows removal only when the eject knob 26 is pressed. In a similar way, additional operating keys such as a record knob (not shown in Figure 2) for transmitting the data from the blood pressure monitor 11 to the base unit may be conceived.

When the patient wishes to have a print out of the medication compliance data and of the health parameter data, he may simply push a printer button 17 on the printer 13 which is separate from the base unit 16. Instead of a button 17 on the printer 13, a like button on the base unit 16 may be provided. When the button 17 is actuated, the printer 13 establishes an infrared communication IR and data are transmitted from the base unit 16 to the printer 13. Data may subsequently be printed out on a paper P.

Different variations of the embodiment shown in Figure 2 are also conceivable. Instead of a printer 13, a personal digital assistant 33 may be used for storing and/or displaying medication compliance data and has parameter data. Although in Figure 2 a printer 13 and a personal digital assistant 33 are shown in parallel, those devices are typically alternatives where either the one or the other is used for display of the data. A personal digital assistant may also be used in combination with the embodiment of Figure 1.

According to a modification of the embodiment shown in Figure 2, it is also conceivable to dispense with the base unit 16 and to use a stand alone medicine container 10 and stand alone blood pressure monitor which both may communicate data directly to a printer or to a personal digital assistant. In this case, both the medicine container 10 and the blood pressure measuring device are provided with memories for storing a series of respective data. Of course, also more than one medicine container may be used in such a case.

The personal digital assistant 33 may be used for different purposes. Data may be displayed on a display of the personal digital assistant 33. The personal digital assistant 33 may also be used to print out the data on a standard printer or to transmit the data over an appropriate communication link such as a mobile phone or another telecommunication link to a central computer within the patient's or the doctor's premises or to an internet server.

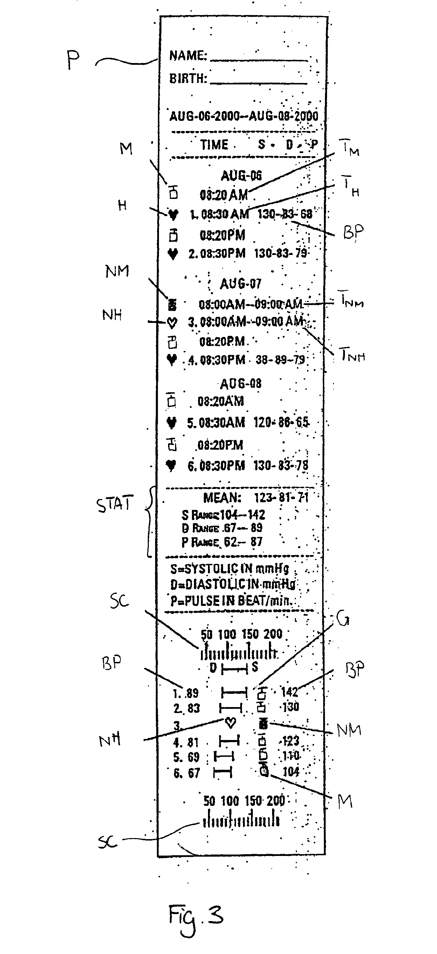

Figure 3 shows an example of a print out made with a system and with a method according to the present invention. The medication compliance data and health parameter data are printed out on a paper strip P. Before the patient meets his doctor, he prints out the data. The paper P is shown to the doctor and may be easily attached to the doctor's patient file. While a print out of the data on a paper P is preferred according to the present invention, other display means, especially display on a computer screen/liquid crystal display are within the scope of the invention. According to the present invention, medicine compliance data and health parameter data may be printed out or displayed in parallel as is shown in Figure 3.

For each patient, a medication schedule and a health parameter measurement schedule is defined. The devices shown in Figure 1 and 2 may be provided with appropriate input interfaces for entering the schedules in a memory contained in the devices. The print out P according to Figure 3 shows data which are printed out for three days. According to the medication schedule, the patient in this example must take his medication twice a day, once in the morning and once in the evening. A time window between 8.00 am and 9.00 am and between 8.00 pm and 9.00 pm is preset. Within this time window, the patient must take its medication and must also perform a health parameter measurement.

On the first day, the patient has complied with the medication schedule and with the health parameter measurement schedule. Compliance with the medication schedule is indicated with a simple M in the form of an empty medicine container. The time of compliance T_{M} with the medication schedule is indicated. The patient has also complied with the health parameter measurement schedule. This is indicated with a symbol H in the form of a bold heart. Next to this symbol H, the time of the health parameter measurement T_{H} and the measurement values BP are displayed.

On the next day (August 7), the patient did not comply with both, the medication schedule and the health parameter measurement schedule. This is indicated with symbols in NM & NH in the form of an empty medicine container/a faint heart. Next to the symbols NM, NH, the time window T_{NM}, T_{NH} within which the patient did not comply with the schedules is displayed.

The example shown in Figure 3 comprises data for three days. Of course, data of longer periods of time may be displayed. Below the daily data, there is the statistical summary STAT of the measurement results. The mean values of the systolic, diastolic blood pressure and of the heart rate as well as the ranges of those values are indicated.

At the bottom of the print out P, there is a graphic representation of the health parameters and of medicine compliance/non compliance. The numeric values BP of the health parameters as well as a graphic representation G in the form of a bar are displayed. Next to the graphic representation G, medicine compliance/non compliance symbols M, NM are displayed. In addition, health parameter measurement non compliance symbols NH are displayed instead of a graphic bar G when the patient did not comply with a measurement schedule. A scale SC helps to determine the values of the health parameters indicated with the graphic G.

On the basis of the information printed out on the bottom of the print out P, the doctor may easily monitor the effectiveness of the treatment, especially the effectiveness of the medication. The doctor immediately sees, that the patient did not comply with the third of the medication schedule and health parameter measurement. Correlation between non compliance with the medication and a change of the health parameter values as indicated for measurement for 4, 5 and 6 may be observed.

According to the present invention, which provides a correlated and comparable print out of medicine compliance data and health parameter measurement data, the effectiveness of a treatment may be easily monitored.

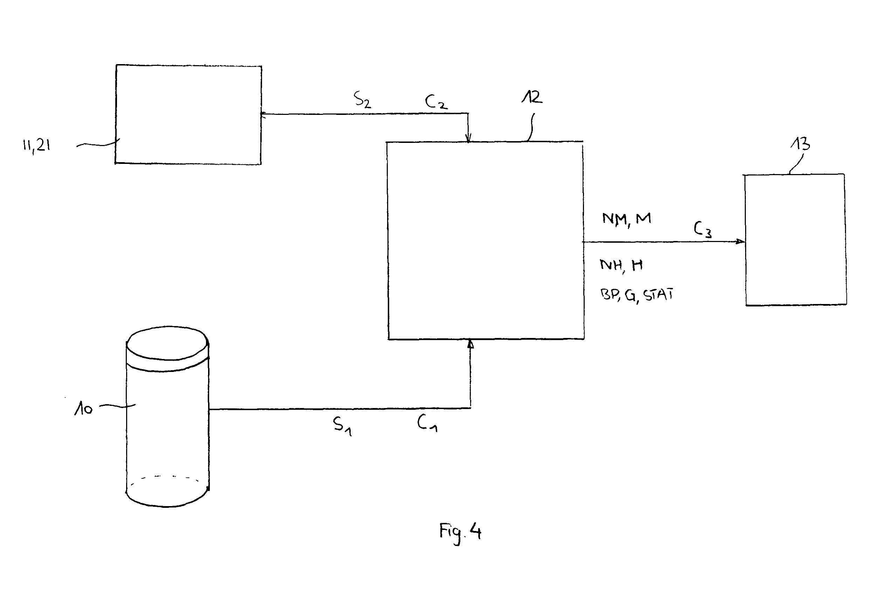

Figure 4 shows a more general schematic representation of the system and the method according to the present invention.
A medicine container 10 generates a first signal S₁ which is indicating removal of medicine from the container 10. The signal is S₁ is transmitted to a control and calculating unit 12 with a communications link C₁.

A health parameter is measured with a health parameter measuring device 11, 21, whereby a second signal S₂ is generated. The second signal S₂ is transmitted to the control and calculating unit 12 with a communications link C₂. Any kind of communications link C₁, C₂ is basically possible: fixed wire connections such as in the embodiment according to Figure 1, partially fixed and partially connectable connections as in the embodiment of Figure 2, wireless connections via e.g. infrared or connections over telecommunication networks are conceivable.

In the control and calculating unit 12, medicine compliance data and health parameter data are calculated and stored. Data such as data indicating compliance M, H or non compliance NM, NH with a prescribed medication or health parameter measurement schedule or measuring results BP or graphic representation G or statistical information STAT may be transmitted over a communications link C3 to a display in the form of a printer 13 or in any other appropriate form. Transmissions between the several parts over the communication links C1, C2, and C3 may be made permanently, on a regular basis or upon request of the user.

Intermediate storage of the data may be made in any of the components shown in Figure 4 e.g. in the medicine container 10, in the measuring device 11, 21 in the control and calculating unit 12 or in the display unit 13.

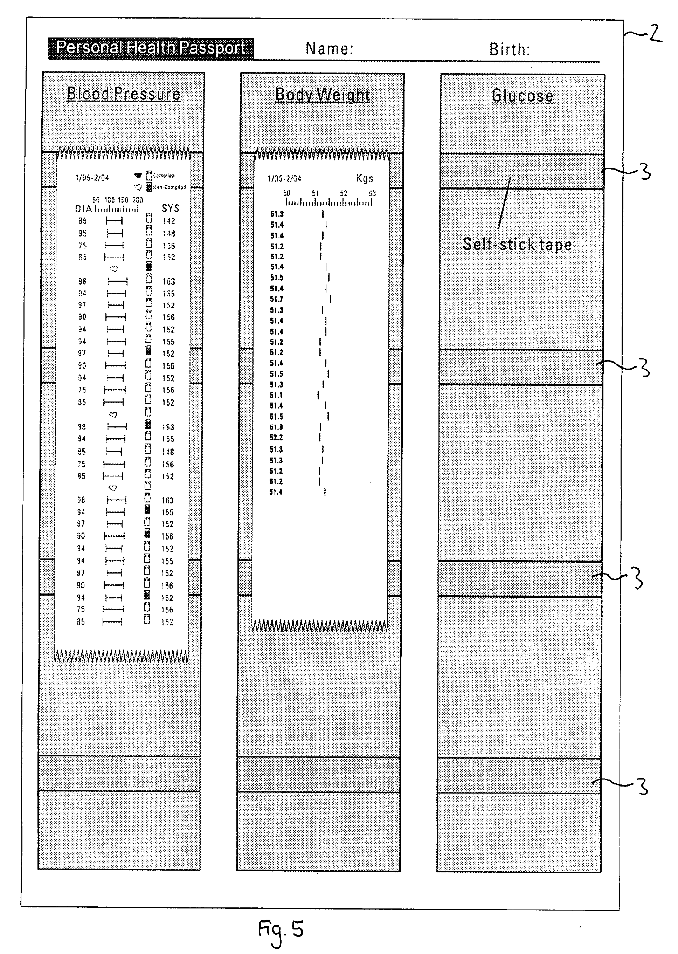

Figure 5 shows a personal health passport obtained with a system according to the invention. A sheet 2 of paper or other suitable material such as cardboard is provided with strips 3, of adhesive tape. Print outs P, P1 relating to medication compliance data and health parameter data may be attached to the strips on appropriate locations on the sheet 2, having labels such as "Blood Pressure", "Body weight" or "glucose". A slightly amended form of print outs as compared to Figure 3 is shown.

The sheet 2 with the print outs, P, P1 may be copied on a photocopier for filing in order to prevent lost of other data printed with a thermo printer in the course of time.

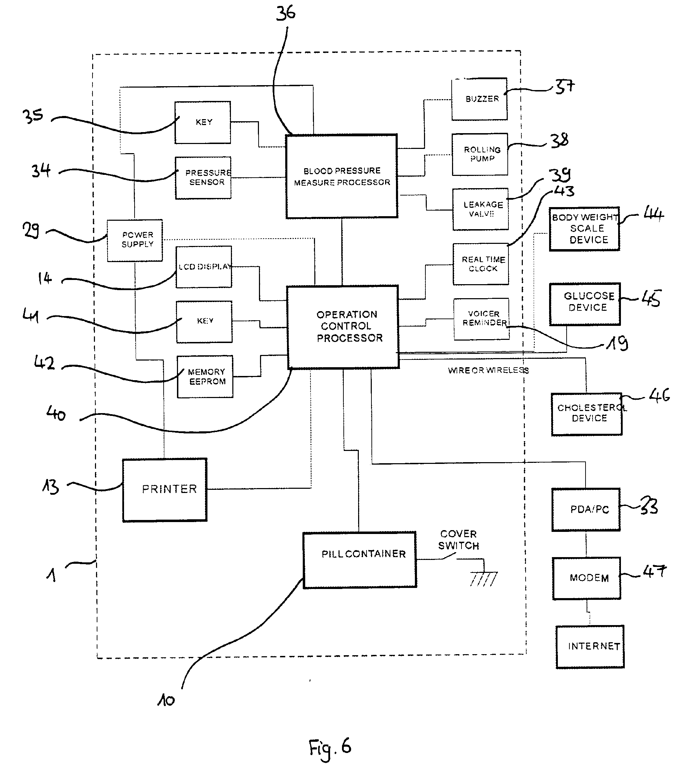

Figure 6 shows a block diagramm of the first embodiment of the invention. The dashed rectangle is surrounding those components which are part of the system 1 according to the first embodiment of the invention (see Figure 1). A central power supply 29 such as a battery is included for operation of all system components. On top of Figure 6, the components of the blood pressure measuring device included in the control and calculating unit are shown. A pressure sensor 34, a blood pressure measurement microprocessor 36 and an operations key 35 are part of the control and calculating unit. A buzzer 37, an inflation pump 38 and a leakage valve 39 are also part of the blood pressure measuring device.

The control and calculating unit further includes a microprocesssor 40 for controlling the operation of the system 1. An LCD display 14 is connected to the microprocessor 40. An operation key 41, a memory 42, a printer 13 and a medicine container 10 are further connected to the microprocessor 40.

An real time clock 43 for operation of the system and a loud speaker 19 are in addition connected to the microprocessor 40.

Optionally, external devices such as a body weight scale 44, a glucose meter 45, a cholesterol meter 46 may be connected to the microprocessor 40 with a wire or a wireless connection. Additionally, a personal digital assistant 33 is connectable to the microprocessor 40. The personal digital assistant 33 may be connected via a modem 47 to the internet. Instead of a personal digital assistant, a personal computer may be connected to the system.

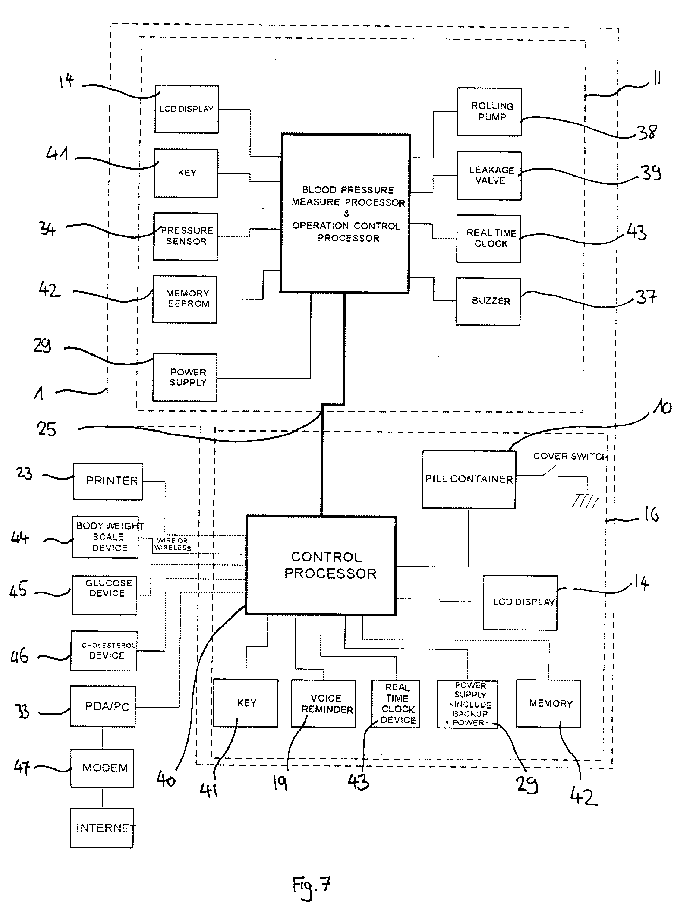

A block diagramm of the embodiment according to Figure 2 is shown in Figure 7. The system according to Figure 7 is different from the system shown in Figure 6, especially in that the blood pressure measurement device is designed as an independently operatable device with separate power supply 29, memory 42, real time clock 43 and display 14. This systems includes a microprocessor which also controls operation of the blood pressure measuring device. The other components are similar to the components shown in Figure 6.

The independently operable wrist type blood pressure measuring device 11 is shown in the upper half of Figure 7. The inner dashed lines define the blood pressure monitor and the base unit respectively. The out dashed line defines the component of the system. In the lower half of Figure 7, the base unit 16 of the second embodiment is shown. The base unit 16 includes a microprocessor 40 for controlling the operation. Similar parts as in Figure 6 are connected to the microprocessor 40. Each of the base unit 16 and of the wrist type blood pressure monitor 11 include parts necessary for independent operations such as a power supply 29, a memory 42, a real time clock 43 and an LCD display 14. The base unit 16 and the blood pressure monitor 11 are connectable with each other via an interface 25. The printer 23 is connectable to the microprocessor 40 with a wire or a wireless connection. Additional devices such as body weight scale 44, glucose meter 45, cholesterol meter 46, a personal digital assistant or a personal computer 33 for connection to the internet via a modem 47 may be connected with a wire or wireless connection to the microprocessor 40.

## Claims

1. A system for monitoring a medical treatment of a patient, the system comprising
- at least one medicine container (10) for storing medicine and designed to produce a first signal (S1) indicative of the patient taking medicine, such as indicating a container opening,
- at least a measuring device (11, 21, 44, 45, 46) for measuring at least one health parameter of the patient for producing a second signal (S2) indicative of said health parameter,
- a control and calculating unit (12) and
- a printer (13, 23) or an other display means (33),
wherein said first signal (S1) may be coupled into said control and calculating unit (12) for storing and/or displaying medication compliance data (14)
wherein said second signal (S2) may be coupled into said control and calculating unit (12) for displaying and/or storing health parameter
and wherein said printer (13) or said other display means (33) is adapted to be coupled to said control and calculating unit in such a way that a series of medication compliance data and health parameter data of the patient may be printed out with said printer (13) or displayed on said display (14; 33).

2. A system according to claim 1, wherein a medication schedule and/or a health parameter measurement schedule is stored in a memory (42) associated with the control and calculating unit (12).

3. A system according to claim 1, wherein the system is formed as an integral unit with a housing (27) including said medicine container (10), said measuring device (21) and said printer (23).

4. A system according to claim 1, wherein the system comprises a base unit (16) wherein the medicine container (10) and/or at least one measuring device (11, 44, 45, 46) is adapted to be connected to and removed from said base unit (16).

5. A system according to claim 4, wherein said printer (13) is designed as a remote printer (13)separate from said base unit (16) and wherein medication compliance data and health parameter data may be transmitted to said printer via a communications link (IR).

6. A system according to claim 1, wherein the system (1) comprises means (17) for causing said printer to print out medication compliance data and health parameter data.

7. A system according to claim 1, wherein the system comprises a voice generator (19) for reminding the patient to take medicine and/or to measure health parameters.

8. A system according claim 1, wherein said control and calculating unit is associated with a memory means (42) for storing a series of subsequent medication compliance data and a series of subsequent health parameter data.

9. A system according to claim 8, wherein said control and calculating unit is programmed in such a way as to calculate a log or chart of a series of successive medication compliance data and health parameter data to be printed out on said printer.

10. A system according to claim 9, wherein said control and calculating unit is programmed in such a way as to indicate compliance of the patient with a medication schedule and/or a health parameter measurement schedule by displaying symbols (NM, NH, M,H ).

11. A system according to one of the claims 1 to 10, wherein the system includes a sheet like personal health passport (2) to which print outs (P, P1) of a plurality of health parameter data and medicine compliance data may be attached, preferably by means of adhesive strips (3) on said passport (2).

12. A method for monitoring a medical treatment of a patient, the method comprising the steps of
a) generating a first signal (S1) indicative of a patient taking a medicine.
b) transmitting said first signal (S1)to a control and calculating unit (12) and storing or displaying medicine compliance data relating to said first signal (S1).
c) measuring at least one health parameter of said patient and generating at least a second signal (S2) indicative of a health parameter of said patient.
d) transmitting said second signal to a control and calculating unit (12) and storing a displaying health parameter data.
e) repeating at least steps a) and b) at least once for generating a series of successive medicine compliance data and a series of successive health parameter data.
f) printing or displaying said series of successive medicine compliance data and health parameter data.

13. A method according to claim 12, comprising the further step of comparing the medicine compliance data and the health parameter data with a medicine compliance schedule and a health parameter measurement schedule stored in a memory (42).

14. A method according to claims 12, comprising the further step of transmitting medicine compliance data and health parameter data via a communications link, especially via a wireless communications link to a printer (13).

15. A method according to claim 14, comprising the further step of generating voice messages for reminding the patient to comply with a medication schedule and/or to measure health parameters.

16. A method according to claim 12, comprising the further step of calculating and displaying statistic values (STAT) of the health parameter data.

17. A method according to claim 12, wherein compliance and non compliance with scheduled medication and/or health parameter measurements is displayed with symbols (NH, NM, H, M.

18. A method according to claim 12, comprising the further steps of displaying a log or chart of a series of successive medication compliance data and health parameter data.

19. A method according to claim 12, comprising the further step of attaching at least one print out (P) produced in step f on a sheet like personal health passport (2).
